(19) Europäisches Patentamt
European Patent Office
Office européen des brevets

(11) Publication number: 0 320 630 A1

# (12) EUROPEAN PATENT APPLICATION

(21) Application number: 88118991.4

(22) Date of filing: 14.11.88

(51) Int. Cl.[4]: C07D 207/09 , A61K 31/40

(30) Priority: 19.11.87 US 122382

(43) Date of publication of application: 21.06.89 Bulletin 89/25

(84) Designated Contracting States: AT BE CH DE FR GB LI NL

(71) Applicant: THE VANDERBILT UNIVERSITY
Box 416
Nashville, TN 37203(US)

(72) Inventor: de Paulis, Tomas
913 Bradford Avenue
Nashville, TN 37204(US)
Inventor: Kessler, Robert M.
1247 Saxon Drive
Nashville, TN 37215(US)
Inventor: Smith, Howard E.
3610 Trimble Road
Nashville, TN 37215(US)
Inventor: Janowsky, Aaron
5700 Southeast Taylor St.
Portland, OR 97214(US)
Inventor: Clanton, Jeffrey A.
308 Cotton Blossom Court
Nashville, TN 37221(US)

(74) Representative: Patentanwälte Grünecker, Kinkeldey, Stockmair & Partner
Maximilianstrasse 58
D-8000 München 22(DE)

## (54) Enantiometric iodobenzamides.

(57) Novel iodine-substituted benzamide derivatives of formula I

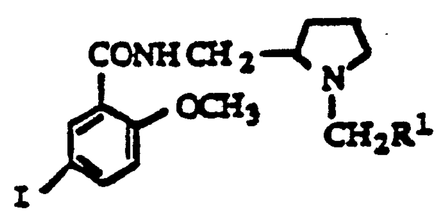

wherein $R^1$ is a hydrogen atom, a lower alkyl group consisting of 1 to 4 carbon atoms, a cycloalkyl group consisting of 3 to 7 carbon atoms, an alkenyl group consisting of 2 to 4 carbon atoms, an alkynyl group consisting of 2 to 4 carbon atoms, a phenyl group, a halogen substituted phenyl group, or an enantiomer or pharmaceutically suitable salt thereof, exhibit high blocking activity and specifity for the dopamine D-2 receptor.

EP 0 320 630 A1

# ENANTIOMETRIC IODOBENZAMIDES

## FIELD OF THE INVENTION

The present invention relates to novel, substituted benzamides which are neuropharmacologically active, their method of preparation, intermediates useful in their method of preparation, and compositions containing the iodobenzamides and their methods of use. More particularly, the present invention relates to novel compounds, substituted benzamide derivatives which have close structural similarities with the structure of (S) and (R)-sulpiride. The neuropharmacological receptor binding profile of the novel compounds mimic that of (RS)-sulpiride, a proven antipsychotic agent and selected blocker of dopamine receptors in the mammalian brain.

Seeman, P: Brain Dopamine Receptors, Pharmacol. Rev., 32:229-313 (1980) reports that the antipsychotic action of neuroleptic drugs against schizophrenia correlates with their ability to block the dopamine D-2 receptor in the mammalian brain. Liskowski, et al.: D-2 Dopamine Receptors in the Frontal Cortex of Rat and Human, Life Sci., 36:1551-1559 (1985) reports that the incidences of drug induced, Parkinson-like side effects (EPS) also seem to correlate with the D-2 receptor blockade determined as displacement of radiolabelled butyrophenones, such as spiperone, from rat brain homogenates. However, according to Magnnusson, et al.: Dopamine D-2 Receptors and Dopamine Metabolism, Neuropharmacol., 25:187-197 (1986) spiperone also binds to receptors in other areas of the rat brain such as the frontal cortex where 1/4 of the binding of [$^3$H] spiperone is selectively bound to the D-2 receptor. Sulpiride, an atypical neuroleptic agent, has less propensity to induce EPS in patients compared with the phenothiazines or butyrophenones in spite of a very high selectivity in blocking only the D-2 receptor. K. Fuxe, et al.: Neurosci. Lett., 64:163-168 (1986) have reasoned that this can be explained as a specific blockade by sulpiride like that of other substituted benzamide neuroleptics to a subpopulation of D-2 receptors, possibly in the hippocampus, but not in the frontal cortex. Thus, there is a need to study the regional distribution and the receptor binding profile of substituted benzamide compounds structurally related to sulpiride. In this regard, the present invention relates to high affinity, D-2 receptor blocking iodobenzamides in which all the structural features of (S)-sulpiride are retained except that the aromatic substitutent in the 5-position (aminosulfonyl) has been replaced by an iodine atom.

U.S. Patent No. 3,342,826 discloses the following compound which is indicated to be an inhibitor of the conditioned avoidance behavior in the rat, a test indicative of dopamine receptor blockade in the central nervous system:

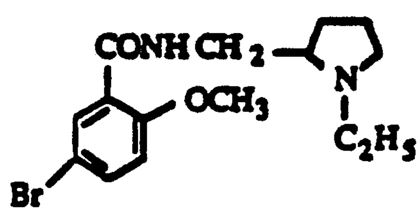

European Patent Application EP 60235 discloses the following compound which is indicated to be an antagonist of the apomorphine syndrome in the rat:

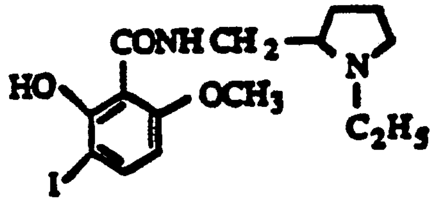

U.S. Patent 4,172,143 discloses the following compound which is indicated to be an antagonist of the apomorphine syndrome in the rat:

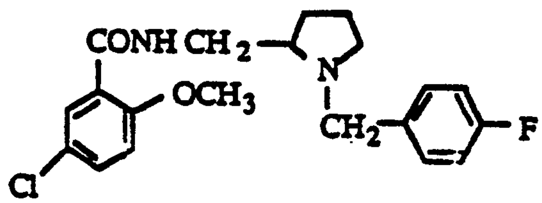

SUMMARY OF THE INVENTION

The present invention relates to substituted benzamides and intermediates useful in their preparation. The compounds are useful for treating emesis and psychosomatic diseases such as depression and schizophrenia, alcoholic related confusional states and sleep disturbances, for they selectively block dopamine D-2 receptors in the mammalian brain. The substituted benzamides are represented by Formula I.

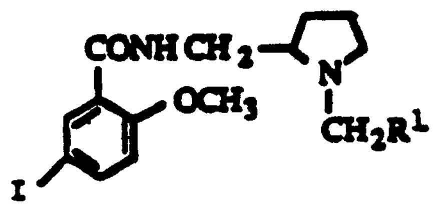

I

Wherein $R^1$ is a hydrogen atom, a lower alkyl group consisting of 1 to 4 carbon atoms, a cycloalkyl group consisting of 3 to 7 carbon atoms, an alkenyl group consisting of 2 to 4 carbon atoms, an alkenyl group consisting of 2 to 4 carbon atoms, a phenyl group, a halogen-substituted phenyl group, or an enantiomer or pharmaceutically suitable salt thereof.

DETAILED DESCRIPTION OF THE INVENTION

The present invention provides substituted benzamide derivatives represented by Formula I:

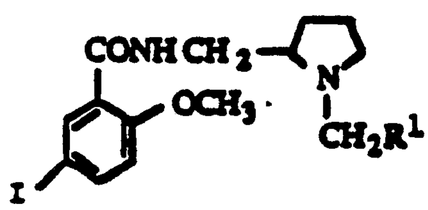

I

wherein $R^1$ is a hydrogen atom, a lower alkyl group consisting of 1 to 4 carbon atoms, a cycloalkyl group

consisting of 3 to 7 carbon atoms, an alkenyl group consisting of 2 to 4 carbon atoms, an alkynyl group consisting of 2 to 4 carbon atoms, a phenyl group, a halogen substituted phenyl group, or an enantiomer or pharmaceutically suitable salt thereof.

The term "pharmaceutically suitable salts" refers to nontoxic acid addition salts of the compounds of this invention which are generally prepared by reacting the free base with a suitable organic or inorganic acid or in situ during the final purification. Representative salts include the hydrochloride, hydrobromide, sulfate, bisulfate, laurate, borate, benzoate, lactate, phosphate, tosylate, citrate, maleate, fumarate, tartrate, napsylate, and the like salts. It would be understood that the persalts are included within the term, i.e., dihydrochloride, etc.

Compounds of Formula I are useful as agents for treating mental disorders such as psychosis and schizophrenia. The agents are potent in their ability to block D-2 receptors in the brain, such as those identified by sulpiride and spiperone. The agents possess a favorable psychochemical profile which endows them with an enhanced distribution across the blood-brain barrier, compared to prior art compounds, which limits the incidences of side effects due to blockage of peripheral dopamine receptors. Thus, for example, compounds of the present invention possess dopamine D-2 receptor blocking activity as evident from the results of a standardized test for their capacity to block sulpiride and spiperone. The procedure, described by de Vries, D.J., and Beart P.M., Eur. J. Pharmacol. 106 133-139 (1984), is as follows: Male Sprague-Dawley rats (150-200 g) were killed by decapitation and their brains quickly removed and dissected on ice. Striata were dissected and homogenized in 40 vol of ice-cold 50 mM Tris-HCl buffer (pH 7.4) containing 120 mM sodium chloride, 5 mM potassium chloride, 2 mM calcium chloride, and 1 mM magnesium chloride using a Brinkman Polytron. The suspension was centrifuged at 30,000 x g for 10 min at 4 °C, and the pellet was resuspended in 40 vol of fresh buffer. The suspension was incubated at 37 °C for 10 min, centrifuged again at 30,000 x g for 10 min at 4 °C, and the final pellet was resuspended with the polytron in 200 vol of ice cold buffer. Assay tubes contained 50 µL of radioligand [$^3$H]spiperone at 0.5 nM and [$^3$H](S)-sulpiride at 5.0 nM concentration, 50 µL of various concentrations of drug in buffer, and 90 µL of the striatal membrane preparation. Nonspecific binding was determined in the presence of 10 µM concentration of unlabeled radioligand. Tubes were incubated for 30 min at 37 °C, poured over Whatman GF/B filters under vacuum, and the filters were rinsed three times with 4 mL of ice cold buffer. Radioactivity remaining on the filters was measured by liquid scintillation on a Quantum 8 Multichannel analyzer operating at 35% efficiency. Table 1 shows the results.

TABLE 1

| Test compound | Blocking of binding IC50 (nM). [3H](S)-sulpiride | [3H]spiperone |
|---|---|---|
| **Prior art compounds:** | | |
| 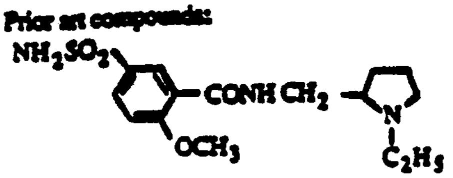 Sulpiride (US 3342826) | 36 ± 3.6 | 332 ± 124 |
| 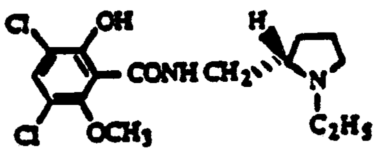 raclopride (EP 60235) | 14 ± 3.5 | 40 ± 20 |
| **Compounds of the invention:** | | |
| 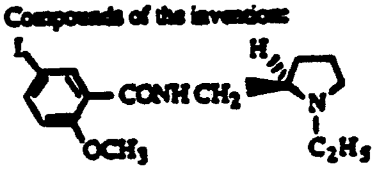 | 145 ± 5 | 560 ± 200 |
| 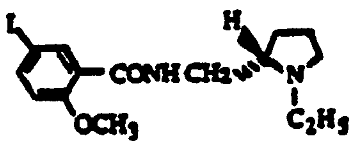 | 3.3 ± 0.9 | 16.4 ± 4.9 |
| 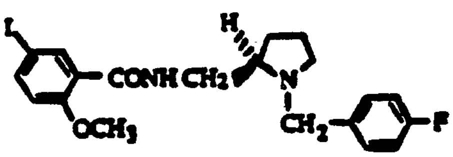 | | |

The results are, of course, specified merely for the purpose of illustration and, accordingly, are not to be construed as either delimiting or exclusionary. Appropriate dosages in any given instance, of course, depend upon the nature of the severity of the condition treated, the route of administration and the species of mammal involved, including its size and any individual idiosyncrasies which obtain.

Suitable daily doses for oral administration of the compounds of this invention are about 1 to about 100 mg.

For therapeutic purposes, the compounds of this invention are ordinarily combined with one or more adjuvants appropriate to the indicated route of administration. The compounds can be utilized in the form of gelatin capsules, tablet, paste, pills, granules and injectable solutions, which are prepared in conventionally known manners. It is possible to use substances which are inert relative to the compounds of the invention such as lactose, magnesium stearate, starch, talc, cellulose, levilite, alkali metal laurylsulfates, saccharose and vehicles conventionally used in medicinal preparations.

In order to prepare tablets, the chosen compound is mixed with starch and lactose by the process of successive dilutions. The mixture is granulated with methyl cellulose. Levilite, magnesium stearate and talc are added to the granular material before tableting is performed.

It is possible to replace the methyl cellulose with any other appropriate granulating agent such as for example, ethyl cellulose, polyvinylpyrrolidone, starch paste, gum arabic etc. The starch can also be replace by different disintegrating agents such as cornstarch, alginates, microcrystalline cellulose etc.

To prepare an injectable solution, it is possible to dissolve the compound according to the invention in aqueous solutions of the following acids: hydrochloric, levullinic, gluconic or glucoheptonic acids. The solution which is prepared in a sterile manner is made isotonic by addition of sodium chloride, after which preservatives are added. It is also possible to prepare the same solution without adding preservatives, for the ampule can be filled under nitrogen and sterilized for 30 minutes at 100° C. The compounds according to the invention can be administered at doses of about 10-50 milligram daily, the preferred daily dosage being about 20 milligram.

Preferred compounds of the present invention include the following:

Formula II

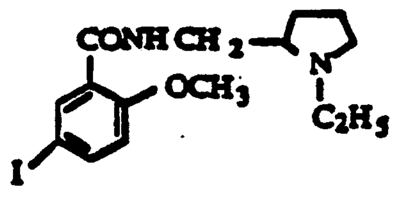

Formula III

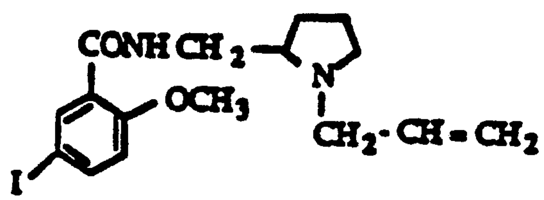

Formula IV

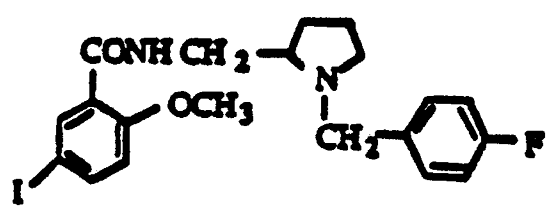

These compounds may be used therapeutically as racemic mixtures or their optically resolved enatiomers. The corresponding enatiomers can be obtained either by fractional crystallization of their salts within optically active acid or by condensation of the appropriately substituted benzoic acid moiety with the enantiomeric forms of 1-alkyl-2-aminomethylpyrrolidine.

Intermediates useful in the preparation of the compounds in Formula I are represented by Formula V:

Formula V

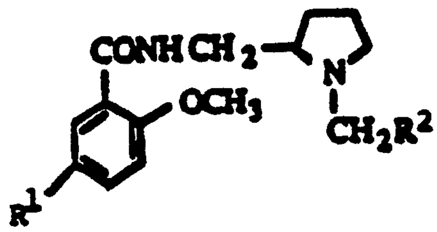

wherein $R^1$ is a trialkylstannyl group such as tri-*n*-butyltin group, 3,3-dialkytriazene moiety, such as 3,3-(1,4-butanediyl)triazene or 3,3-(1,5-pentanediyl)triazene moiety or an amino group; $R^2$ is a hydrogen atom, a lower alkyl group consisting of 1 to 4 carbon atoms, a cycloalkyl group consisting of 3 to 7 carbon atoms, an alkenyl group consisting of 2 to 4 carbon atoms, an alkynyl group consisting of 2 to 4 carbon atoms, a phenyl group, a halogen-substituted phenyl group, or an enantiomer or pharmaceutically suitable salt thereof.

Compounds of the present invention can be prepared by any one of the following methods:

A. Compounds of Formula I can be obtained by the reaction of the intermediate compound of formula V, wherein $R^1$ equals a tributyltin group, with iodine in a protic solvent such as ethanol or hydrochloric acid. The iodine can be generated *in situ* by decomposition of iodine monochloride or by oxidation of the alkali metal iodide with hydrogen peroxide or an agent such as the sodium salt of *N*-chloro-*P*-toluenesulfonamide.

B. Compounds of Formula I can be obtained by the reaction of the intermediate compound of formula V, wherein $R^1$ is a triazeno group of the formula

R
N-N=N-
R

where R is a lower alkyl group of 1 to 4 carbon atoms or constitutes a 5- or 6-member ring with an acid, such as hydrochloric acid or trifluoroacetic acid, in the presence of iodide in a solvent, such as acetone, formic acid or acetonitrile.

C. The compounds of Formula I can be obtained by the reaction of 5-iodo-2-methoxybenzoyl chloride with a 1-alkyl-, 1-allyl, 1-cycloalkylmethyl-, or 1-(4-substituted-benzyl)-2-aminomethylpyrrolidine in a aprotic solvent such as methylene chloride, chloroform, or acetonitrile at room temperature.

D. The compounds of Formula I can be prepared by the reaction of an intermediate compound below with an alkylating agent such as ethyl iodide, allyl bromide, cyclopropyl halide, or *p*-fluorobenzyl bromide in a solvent such as dimethylformamide in the presence of a base such as potassium carbonate or pyridine.

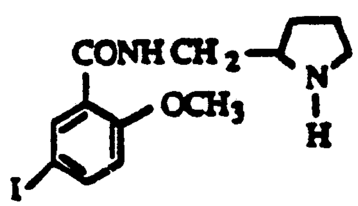

E. The compounds of Formula I can be prepared by reacting the intermediate compound below with iodine or iodine monochloride in a solvent such as dioxane, tetrahydrofuran or chloroform, or in mixtures thereof.

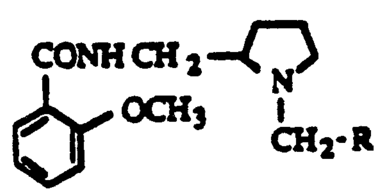

The following examples describe in detail compounds illustrative of the present invention and methods which have been devised for their preparation.

EXAMPLE 1.

(S)-N-[(1-Ethyl-2-pyrrolidinyl)methyl]-5-iodo-2-methoxybenzamide. (Method A).

Sodium iodide (0.15 g, 0.001 mol) was dissolved in 0.3 N hydrochloric acid (15 mL) and added to a solution of (S)-5-tri-n-butyltin-N-[(1-ethyl-2-pyrrolidinyl)methyl]-2-methoxybenzamide( 0.67 g, 0.001 mol) in ethanol (2 mL). A solution of sodium N-chloro-4-methylbenzenesulfonamide (0.27 g, 0.0012 mol) in water (12 mL) was added at 20 °C The solution turned dark then colorless. After stirring at 20 °C. for 1 h additional 0.3 N HCl (100 mL) was added, and the reaction mixture was shaken with ether (100 mL),, the aqueous layer was neutralized by addition of 5 N NaOH (20 mL), and the product was extracted with ether (2 x 200 mL). Drying ($Na_2SO_4$) and evaporation of the solvent gave 0.22g (57%) of the desired iodobenzamide as an oil $[\alpha]_D^{20}$ -62° (c 2.01, acetone);NHR:δ 8,43 (d, 1 H), 7.71 (dd, 1H), 6.76 (d, 1 H, J 8.7 $H_z$) 3.97 (s, 3 H), 1.5-3.8 (m, 11 H) 1.13 ppm (t, 3 H).

EXAMPLE 2.

(S)-N-[(1-Ethyl-2-pyrrolidinyl)methyl]-5-iodo-2-methoxybenzamide. (Method B).

A solution of sodium iodide (0.05 g, 0.3 mmol) and trifluoroacetic acid (0.03 g, 0.3 mmol) in 97% formic acid (0.50 mL) was slowly added to a stirred solution of (S)-N-[(1-ethyl 2-pyrrolidinyl)methyl]-2-methoxy-5-[3,3-(1,5-pentanediyl)triazeno] benzamide (0.11 g, 0.3 mmol) in chloroform (2.0 mL) at 20 °C. The mixture was stirred for 45 min. Sodium hydroxide (1 N, 20 mL) was added, and the product was extracted with methylene chloride (3 x 25 mL). Washing of the combined organic layer with water (25 mL), drying ($Na_2SO_4$) and evaporation of the solvent gave 0.14g (60%) of the iodobenzamide as an oil. Possible contamination of the corresponding and simultaneously formed desiodo derivative is avoided by addition of one half equivalent of iodine to the reaction mixture, cf. Coffen et al.: J. Org. Chem., 49:296-300 (1984).

EXAMPLE 3.

(R)-N-[(1-Ethyl-2-pyrrolidinyl)methyl]-5-iodo-2-methoxybenzamide. (Method C).

The di-L-(+)-tartrate salt of (R)-2-aminomethyl-2-ethyl-pyrrolidine (2.0 g, 4.7 mmol) was dissolved in 4 N NaOH (10 mL) and the free amine was extracted with $CH_2Cl_2$ (2 x 20 mL). The volume of the combined extracts was reduced to 20 mL and added dropwise to a solution of 2-methoxy-5-iodobenzoyl chloride 1.1 g, 3.6 mmol), prepared from 2-methoxy-5-iodobenzoic acid, in $CH_2Cl_2$(20 mL) at 30°C. Stirring was continued for 35 min. The solvent was evaporated, and the residue was treated with 1 N NaOH (50 mL). Extraction with ether (3 x 50 mL), extraction of the combined ether solutions with 1 N HCl (2 x 50 mL), neutralization of the combined aqueous layer with 4 N NaOH (30 mL), extraction with $CH_2Cl_2$(3 x 30 mL), and finally drying ($MgSO_4$) and evaporation of the solvent gave 1.3 g of product as an oil: $[\alpha]_D^{20}$ +63° (c 1.63, acetone).
The oil was dissolved in acetone (12 mL) and 3 N HCl-ether (115 mL) and water (one drop) were added. Cooling to 0 °C precipitated 1.2 g (75%) of the iodobenzamide hydrochloride hydrate. Recrystallization

from 98% aqueous acetone (50 mL) gave 0.72 g of the salt: mp 181-182 °C. Anal. ($C_{15}H_{21}IN_2O_2$•HCl•$H_2O$): calcd C 41.54%, found 41.33%; calcd H 5.34%, found 5.52%; calcd Cl 8.17%, found 8.55%; calcd I 29.26%, found 28.98%; calcd N 6.46%, found 6.35%.

EXAMPLE 4.

(R)-N-[(1-(4-fluorobenzyl)-2-pyrrolidinyl)methyl] 5-iodo-2-methoxybenzamide (Method C)

D-Glutamic acid (10g, 0.068 mol) was dissolved in 2 N NaOH (50 ml, 0.10 mol). p-Fluorobenzaldehyde (10g, 0.081 mol) was added at 25 °C and after 30 min $NaBH_4$ (2.0 g, 0.053 mol) was added in portions at 25 °C. After 2 h the reaction mixture was shaken with 50 mL ether to remove unreacted aldehyde. The aqueous layer was neutralized with conc HCl which gave a white precipitate. Heating of the solution to reflux for 2 h, then cooling gave 7.4g (46%) of (R)-N-(4-fluorobenzyl)-2-pyrrolidinone-5-carboxylic acid. This acid was treated with an excess of thionyl chloride in methanol to give the corresponding methyl ester. The latter was dissolved in methanol (30 mL) and treated with ammonia gas for 30 min.
Evaporation of the solvent gave 2.4 g (71%) of (R)-1-(4-fluorobenzyl)-2-pyrrolidinone-5-carboxamide: mp 175-178 °C on recrystallization from ethanol. This amide was suspended in ether (50 mL) and lithium aluminum hydride (1.3 g, 0.034 mol) was slowly added under nitrogen atmosphere. The mixture was heated under reflux for 18 h. After cooling, saturated sodium sulfate (5 mL) was slowly added and the inorganic salts were removed by filtration. Evaporation of the ether gave 1.31g (63%) of (R)-2-aminomethyl-1-(4-fluorobenzyl)pyrrolidine: $[\alpha]_D + 45^\circ$ (c3.9, MeOH).

The diamine (0.70g, 3.3 mmol) was dissolved in $CH_2Cl_2$ (20 mL) and this solution was added to a solution of 5-iodo-2-methoxybenzoyl chloride (0.85g, 2.9 mmol) in $CH_2Cl_2$ (20 mL). The mixture was stirred for 2 h at 25 °C. The solvent was evaporated. The residue was treated with ether (50 mL) and the product was extracted with 1 N HCl (3 x 100 mL). Neutralization of the combined aqueous layer with 30% KOH, and extraction with ether (3 x 100 mL) gave 0.72g of the iodobenzamide as an oil:$[\alpha]_D^{25}$ +56° (c 1.8, MeOH). The oil was dissolved in ethyl acetate (10 mL) and 3 N HCl-ether (1 mL) was added. Filtration gave 0.31 g, of the hydrochloride: mp 118-120 °C. Anal. ($C_{20}H_{22}FIN_2O_2$.HCl. $H_2O$):calcd C 45.95%, found 45.88%; calcd H 4.82%, found 4.91%; calcd Cl 6.78%, found 6.38%; calcd F 3.63%, found 3.47%; calcd I 24.27%, found 23.39%.

EXAMPLE 5.

(S)-N-[1-(4-fluorobenzyl)-2-pyrrolidinyl)methyl]-5-iodo-2-methoxybenzamide. (Method D).

To a solution of (S)-5-iodo-2-methoxy-N-(2-pyrrolidinylmethyl)benzamide (1.4 g, 0.004 mol) in dimethylformamide (20 mL) was added anhydrous potassium carbonate (1.4g, 0.01 mol) followed by dropwise addition of p-fluorobenzyl bromide (0.6 mL, 0.005mol) in dimethylformamide (10 mL) at 20 °C. After stirring for 30 min at 35 °C water was added (100 mL), and the product was extracted with ether (2 x 100 mL). Drying and evaporation of the solvent gave 2.0 g of crude iodobenzamide as an oil: $[\alpha]_D^{25}$ -63° (c 1.7, $CHCl_3$). The oil was dissolved in ethyl acetate (20 mL) and 3 N HCl-ether (10 mL) was added. This gave 0.7 g of the hydrochloride mp:115-118 °C.

EXAMPLE 6.

(S)-N-[(1-Ethyl-2-pyrrolidinyl)methyl]-5-iodo-2-methoxybenzamide. (Method E).

o-Anisic acid (3.0 g, 0.020 mol) was converted to its acid chloride, and the latter was mixed with (S)-2-aminomethylethylpyrrolidine (3.8 g, 0.03 mol) in methylene chloride (30 mL). Stirring was continued for 2 h at 20 °C. Sodium hydroxide (1 N, 30 mL) was added, and the product was extracted with $CH_2Cl_2$ (2 x 30 mL). Washing with water (30 mL), drying ($Na_2SO_4$) and evaporation of the solvent gave the N-substituted anisamide as a colorless oil. Iodine (3.8 g, 0.015 mol) was dissolved in $CHCl_3$ (10 mL) and added to a stirred mixture of the thus prepared (S)-2-methoxy-N-[(1-ethyl-2-pyrrolidinyl)methyl]benzamide (2.6 g, 0.010 mol) in dioxane (20 mL) and $CHCl_3$ (30 mL). The mixture was heated to 80 °C for 4 h and then water (100 mL) was added. Extraction with ether (3 x 50 mL) gave, after purification through a silica column, 2.8 g of iodobenzamide as an oil. The oil was dissolved in 95% aqueous acetone (15 mL) and 3 N HCl-ether (2 mL) was added dropwise and filtration after 2 h gave 1.9 g (43%) of the hydrochloride monohydrate salt: mp 179-181 °C.

EXAMPLE 7.

(Intermediates).

(S)-5-Amino-2-methoxy-N-[(1-ethyl-2-pyrrolidinyl)methyl]benzamide.

(S)-N-[(1-Ethyl-2-pyrrolidinyl)methyl]-2-methoxy-5-nitrobenzamide (3.7 g, 0.011 mol) was dissolved in methanol (120 mL) and under a nitrogen atmosphere, and 10% Pd on carbon (0.5 g) was added.

Hydrogenation at room temperature and one atmosphere pressure consumed the theoretical amount of $H_2$ (740 mL, 0.033 mol) in 1.5 h. The catalyst was removed by filtration through celite and the solvent was evaporated. Ether extraction gave 2.9 g of residue as an oil: $[\alpha]_D^{20}$ -55° (c 0.22, MeOH). The oil was dissolved in hot ethanol (65 mL), and concentrated HCl (1.0 mL) was added. Cooling and addition of ether (25 mL) gave 2.48 g (66%) of the monohydrochloride of the aminobenzamide: mp 217-218 °C, NMR$\delta$ 8.46 (b, NH), 7.56 (dd, 1 H), 6.77 (m, 3.85 (s, 3 H, $OCH_3$), 3.60 (s, 2 H, $NH_2$) 3.7-1.5 (m, 11 H), 1.11 ppm (t, 3 H, $CH_3$). Anal. ($C_{15}H_{23}IN_3O_2$•HCl); calcd C 57.4%, found 57.2%; calcd H 7.71%, found 7.66%; calcd Cl 11.30%, found 10.80%; calcd N 13.39%, found 13.34%.

EXAMPLE 8.

(S)-N-[(1-Ethyl-2-pyrrolidinyl)methyl]-2-methoxy-5-[3,3-(1,4-butanediyl)triazene]benzamide.

The hydrochloride of the aminobenzamide from example (2.2 g, 6.3 mmol) was dissolved in a mixture of 1 N HCl (20 mL) and acetone (10 mL). Sodium nitrite (0.7 g, 0.1 mol) was slowly added at 0 °C. After 15 min, a solution of pyrrolidine (1.5 mL, 0.018 mol) in 50% aqueous acetone (10 mL) was added, and the mixture was stirred at 10 °C for 1.5 h.

Dilute NaOH (60 mL) was added, and the product was extracted with $CH_2Cl_2$(3 x 50 mL). Washing of the combined extracts with water (50 mL), drying ($Na_2SO_4$) and evaporation of the solvent gave 2.5 g of a tan residue. Column chromatography on silica (180 g Davisil 62) in ethyl acetate-ethanol-concentrated ammonium hydroxide (100:10:1) as eluent, and evaporation of the fractions containing a spot at Rf 0.24 on TLC with the same solvent system gave 1.88 g (83%) of the triazene derivative as a viscous oil. High resolution MS, found: m/z 359.2322. Calculated for ($C_{19}H_{29}N_5O_2$):M+ 359.2323. NMR of aromatic protons: 8.23 (d, 1, C(6)-H), 7.44 (dd, 1, C(4)-H), 6.93 ppm (d, 1, C(3)-H).

EXAMPLE 9.

(S)-5-(tri-n-butyltin)-N-(1-ethyl-2-pyrrolidinyl)methyl]-2-methoxybenzamides

(S)-5-Bromo-N-[(1-ethyl-2-pyrrolidinyl)methyl]-2-methoxybenzamide (1.0 g, 2.9 mmol) was dissolved in triethylamine (50 mL), freshly distilled from calcium hydride. Palladium (II) acetate (0.07g, 0.3 mmol), and tetrakis(triphenylphosphine)palladium(0) (0.12 g, 0.1 mmol) were added under a nitrogen atmosphere at 20 °C. Bis(tri-n-butyltin) (3.0 mL, 6.0 mmol) was added, and the mixture was heated at 100 °C. for 3 h. The solution was filtered, and the solvent was evaporated.
The residual oil was subjected to flash chromatography on silica. Elution with hexane removed the excess tributyltin reagent and the remaining triethylamine. Elution with ethyl acetate-ethanol (10:1) gave fractions with a single spot at $R_f$ 0.45 on TLC. These fractions were combined and evaporation of the solvent gave 0.9 g (48%) of desired organotinbenzamide as an oil: NMR: 8.26 (d, 1 H), 7.51 (dd, 1 H), 6.94 (d, 1 H), 3.93 (s, 3 H) 2.6-4.0 (m, 7 H), 2.0-2.6 ppm (m, 34 H).

EXAMPLE 10.

(S)-5-Iodo-2-methoxy-N-2-pyrrolidinylmethyl]benzamide.

N-Tritylprolinamide (4.0 g, 0.023 mol) was dissolved in 30 mL THF and this solution added dropwise to a mixture of lithium aluminum hydride (1.0 g, 0.035 mol) in 15 mL of diethyl ether at 20 °C. After the initial foaming had subsided, the reaction mixture was heated to 45 °C for 30 h. Ether (50 mL) was added followed by dropwise addition of a saturated solution of $Na_2SO_4$ (8 mL). The inorganic salts were removed by filtration,and the solvent was removed by evaporation. Crystallization of the residual viscous oil (3.6 g) from isopropylether (50 mL) gave 3.0 g (75%): mp 106-107 °C.

The pyrrolidine (3.0 g, 0.008mol) was dissolved in $CH_2Cl_2$ (20 mL) and 5-iodo-2-methoxybenzoyl chloride (2.3 g, 0.008 mol) in 30 mL of $CH_2Cl_2$ was slowly added at 23 °C. After stirring for 30 min the solvent was evaporated, and the residue was treated with a mixture of 1.0 mL (0.012 mol) of conc. HCl in 30 mL of ethanol at room temperature for 1 h. Water (200 mL) was added and the mixture was shaken with ether (2 x 100 mL) for the removal of the triphenylcarbinol. The aqueous layer was made alkaline (pH 10) by addition of concentrated $NH_4OH$. Extraction with $CH_2Cl_2$ (4 x 100 mL), washing, drying and evaporation of the solvent gave 1.6 g (56%) of the benzamide as an oil: $[\alpha]_D^{25}$ +27° (c 5.1, $CHCl_3$) The latter was dissolved in 15 mL acetone and addition of 3 N HCl-ether (3 mL) was added gave 1.06 g of the hydrochloride: mp 180 °C dec.

EXAMPLE 11.

| Gelatine Capsules (one 200 mg capsule) | |
|---|---|
| (S)-N-[1-Ethyl-2-pyrrolidinyl)methyl]-5-iodo-2-methoxybenzamide hydrochloride hydrate | 50 mg |
| Dry potato starch | 30 mg |
| Lactose | 113 mg |
| Methyl Cellulose 1500 | 1-6 mg |
| Talc | 2.7 mg |
| Magnesium Stearate | 2.7 mg |
| For a 50 mg capsule | |

EXAMPLE 12.

| Injectable Solution (1 mL of solution) | |
|---|---|
| (S)-N-[1-Ethyl-2-pyrrolidinyl)methyl]-5-iodo-2-methoxybenzamide hydrochloride hydrate | 50 mg |
| Sodium Chloride | 4 mg |
| Water of injectable preparation | 1 mL |
| for a 50 mg solution | |

## Claims

1. An iodobenzamide represented by the formula

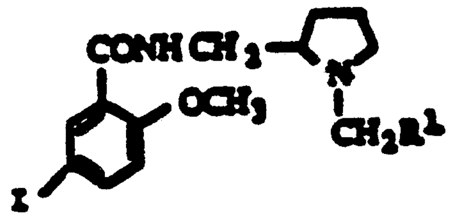

wherein $R^1$ is a hydrogen atom, a lower alkyl group consisting of 1 to 4 carbon atoms, a cycloalkyl group consisting of 3 to 7 carbon atoms, an alkenyl group consisting of 2 to 4 carbon atoms, an alkynyl group consisting of 2 to 4 carbon atoms, a phenyl group, a halogen-substituted phenyl group, or an enantiomer or pharmaceutically suitable salt thereof.

2. A compound of claim 1 wherein $R^1$ is a methyl group.
3. A compound of claim 1 wherein $R^1$ is a p-fluorophenyl group.
4. A compound of claim 1 in the form of optical isomers thereof.
5.(S)-N-[(1-ethyl-2-pyrrolidinyl)methyl]-5-iodo-2-methoxybenzamide.
6. (R)-N-[(1-ethyl-2-pyrrolidinyl)methyl]-5-iodo-2-methoxybenzamide.
7. (R)-N-[(-1-(4-fluorobenzyl-2-pyrrolidinyl)methyl]-5-iodo-2-methoxybenzamide.
8. (S)-N-[(1-(4-fluorobenzyl-2-pyrrolidinyl)methyl]-5-iodo-2-methoxybenzamide.
9. (S)-N-[1-allyl-2-pyrrolidinyl)methyl]-5-iodo-2-methoxybenzamide.
10. A substituted benzamide intermediate represented by the formula:

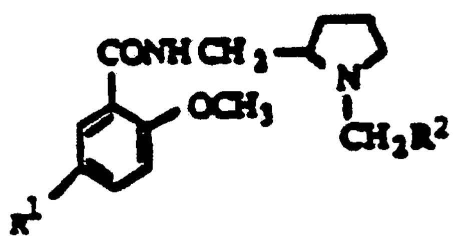

wherein $R^1$ is a trialkylstannyl group such as tri-n-butyltin, a 3,3-dialkytriazene moiety, such as 3,3-(1,4-butanediyl)triazene or 3,3-(1,5-pentanediyl)triazene moiety or an amino group; $R^2$ is a hydrogen atom, a lower alkyl group consisting of 1 to 4 carbon atoms, a cycloalkyl group consisting of 3 to 7 carbon atoms, an alkenyl group consisting of 2 to 4 carbon atoms, an alkynyl group consisting of 2 to 4 carbon atoms, a phenyl group, a halogen-substituted phenyl group, or an enantiomer or pharmaceutically suitable salt thereof.

11. The compound of claim 10 wherein $R^1$ is a tri-n-butyltin group.
12. A pharmaceutical composition comprising an effective amount of at least one compound according to any of claims 1 to 9.

European Patent Office

# EUROPEAN SEARCH REPORT

Application Number

EP 88 11 8991

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 305 973 (YAMANOUCHI PHARMACEUTICAL) * Claim 1, page 2 * --- | 1,12 | C 07 D 207/09<br>A 61 K 31/40 |
| P,X | CHEMICAL ABSTRACTS, vol. 109, no. 9, August 1988, page 49, no. 66715n, Columbus, Ohio, US; A. JANOWSKY et al.: "Iodopride: a specific high affinity radioligand for labeling striatal dopamine D-2 receptors", & EUR. J. PHARMACOL. 1988, 150(1-2), 203-5 * Abstract * --- | 1,12 | |
| P,X | JOURNAL OF MEDICINE CHEMISTRY, vol. 31, no. 5, May 1988, pages 1039-1043, American Chemical Society; H.F. KUNG et al.: "Dopamine D-2 receptor imaging radiopharmaceuticals: synthesis, radiolabeling, and in vitro binding of (R)-(+)- and (S)-(-)-3-iodo-2-hydroxy-6-methoxy-N-[(1-ethyl-2-pyrrolidinyl)meth]benzamide" * Whole article * --- | 1,12 | **TECHNICAL FIELDS SEARCHED (Int. Cl.4)** |
| P,X | JOURNAL OF MEDICINAL CHEMISTRY, vol. 31, no. 10, October 1988, pages 2027-2033, American Chemical Society; T. DE PAULIS et al.: "(S)-N-[(1-ethyl-2-pyrrolidinyl)methyl]-5-[125I]iodo-2-methoxybenzamide hydrochloride, a new selective radioligand for dopamine D-2 receptors" * Whole article * --- -/- | 1,12 | C 07 D 207/00<br>A 61 K 31/00 |

The present search report has been drawn up for all claims

| Place of search | Date of completion of the search | Examiner |
|---|---|---|
| THE HAGUE | 24-02-1989 | CASADO Y MARTIN DE MERCA |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 88 11 8991

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-2 013 662 (SOCIETE D'ETUDES SCIENTIFIQUES ET INDUSTRIELLES DE L'LLE-DE-- FRANCE) * Claims 1,62 * --- | 1,12 | |
| X | GB-A-2 016 467 (SOCIETE D'ETUDES SCIENTIFIQUES ET INDUSTRIELLES DE L'LLE-DE-FRANCE) * Claim 1; page 4 * --- | 1,12 | |
| Y | EP-A-0 105 599 (ADRIA LABORATORIS INC.) * Claims 6,7 * --- | 1,12 | |
| Y,D | EP-A-0 060 235 (ASTRA LÄKEMEDEL) * Claims 1,14 * ----- | 1,12 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 24-02-1989 | CASADO Y MARTIN DE MERCA |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)